# EUROPÄISCHE PATENTSCHRIFT

(11) **EP 0 663 214 B1**
(45) Veröffentlichungstag und Bekanntmachung des Hinweises auf die Patenterteilung: **31.03.1999**
(21) Anmeldenummer: 94100658.7
(22) Anmeldetag: 18.01.1994
(51) Int. Cl.: A61L 2/06, A61L 2/26

(54) **Anordnung zur Dampf-Sterilisation von ärztlichen Instrumenten, Implantaten und ähnlichem**
Assembly for the steam sterilization of surgical instruments, implants and the like
Dispositif pour la stérilisation par la vapeur d'instruments chirurgicaux, d'implants et similaires

(43) Veröffentlichungstag der Anmeldung: 19.07.1995
(73) Patentinhaber: SCI-CAN, A DIVISION OF LUX AND ZWINGENBERGER LTD., Toronto, Ontario M2J 1RJ (CA)
(72) Erfinder: Zwingenberger, Arthur, Toronto, Ontario M4W 2R6 (CA); Saupe, Martin, D-63075 Offenbach (Main) (DE)
(74) Vertreter: Puschmann, Heinz H.

(56) Entgegenhaltungen:
- EP-A- 0 532 135
- DE-A- 4 233 051

## Beschreibung

Die Erfindung betrifft eine Anordnung zur Dampf-Sterilisation von ärztlichen Instrumenten und Implantaten und ähnlichem, mit einem von einer Dosierpumpe gespeisten Dampfgenerator, einem Sterilisationsgefäß, das in einer mit dem Dampfgenerator in Wirkverbindung stehenden Haltevorrichtung einschiebbar angeordnet ist und mit einer Steuereinrichtung für den Sterilisationsvorgang, wie er in der EP 0 429 960 A2 beschrieben und dargestellt ist.

Bei der bekannten Anordnung ist das Sterilisationsgefäß als lose Kassette ausgebildet, deren Boden- und Deckelteil über flanschartige Ränder unter Zwischenfügung einer Dichtung während des Sterilisationsvorganges in der Haltevorrichtung dicht zusammengepreßt gehalten sind.

Die Ausbildung des Sterilisationsgefäßes als zweiteilige, allseitig gegenüber Atmosphäre geschlossene Kassette hat sich jedoch in der Herstellung als aufwendig erwiesen. Ferner sind enge Toleranzen bei der Herstellung einzuhalten, da beim Einschieben der Kassette in die Haltevorrichtung ein Kuppeln mit den zugehörigen Zu- und Ableitungen erforderlich ist.

Aufgabe der Erfindung ist es daher, ein fertigungstechnisch einfacher ausgebildetes und das Beschicken mit Instrumenten und Implantaten, sowie das Bedienen der Anordnung erleichternden Aufbau aufweisendes Sterilisationsgefäß zu schaffen.

Diese Aufgabe ist gemäß der Erfindung dadurch gelöst, daß die Wandungen des während des Sterilisationsvorganges allseits druckdicht geschlossenen Sterilisationsgefäßes zumindest teilweise Teile der Haltevorrichtung sind.

Nach einem weiteren Merkmal der Erfindung ist die Ausbildung derart getroffen, daß das Sterilisationsgefäß auch während des Beschickens mit den zu sterilisierenden Instrumenten und Implantaten mit der zugeordneten Haltevorrichtung betrieblich verbunden bleibt.

Es kann aber auch für Wartungsarbeiten ganz herausgezogen werden, z. B. zum Wechseln von Dichtungen.

Weitere Merkmale der Erfindung ergeben sich aus den Unteransprüchen.

Der verblüffend einfache Grundgedanke der Erfindung, zumindest teilweise Wandungen des während des Sterilisationsvorganges allseits druckdicht geschlossenen Sterilisationsgefäßes der das Sterilisationsgefäß während des Sterilisationsvorganges aufnehmenden Haltevorrichtung zuzuordnen, ist nach einer ersten Ausführungsform der Erfindung dadurch gelöst, daß dem Sterilisationsgefäß lediglich die Vorder- und die Rückwand zugeordnet sind, während die Seiten- sowie die Ober- und Unterwandung der Haltevorrichtung zugeordnet sind, wobei mindestens eine, die Vorder- und Rückwand miteinander verbindende Lochplatte der Aufnahme der zu sterilisierenden Instrumente und Implantate dient. Durch die der Vorder- und Rückwand jeweils zugeordneten Dichtungsmanschetten, die im eingeschobenen Zustand des Sterilisationsgefäßes den von den genannten Wänden eingeschlossenen Raum gegenüber Atmosphäre abdichten, wird gleichzeitig das Sterilisationsgefäß in seiner Wirklage innerhalb der Haltevorrichtung gehalten. Die in den Sterilisationsgefäßraum mündende Dampfzuleitung sowie die in diesen Raum mündende Luft- und Kondensat-Abführleitung sind dabei in der Haltevorrichtung fest installiert, so daß ein "Einfädeln" oder "Einkuppeln" dieser Zu- und Ableitungen in entsprechende Öffnungen innerhalb der Kassette nunmehr entfällt. Die mit der Haltevorrichtung verschiebbar verbundene Führungsvorrichtung wirkt als Stützvorrichtung, die nicht nur ein Abkippen des Sterilisationsgefäßes beim Herausbewegen aus der Haltevorrichtung verhindert, sondern gleichzeitig auch als Auffangwanne für abtropfende Feuchtigkeit der den Sterilisationsraum verlassenden ärztlichen Instrumente und Implantate ausgebildet ist. Durch die auf das Sterilisationsgefäß in der Haltevorrichtung wirkende Druckfeder wird ohne Zutun der Bedienungsperson das Sterilisationsgefäß nach Beendigung des Sterilisationsvorganges aus der Haltevorrichtung in die Offenlage zur Entnahme der sterilisierten Instrumente und zur Wiederbeschickung selbsttätig bewegt. Für das Auslösen dieses Vorganges dient die entweder druck- oder elektromagnetisch betätigte Verriegelungsvorrichtung, die ebenfalls überaus einfach ausgebildet ist, da sie lediglich dem auf das Sterilisationsgefäß ausgeübten Federdruck standzuhalten hat. Während des Sterilisationsvorganges herrschen an den Innenseiten der Vorder- und Rückwandung des Sterilisationsgefäßes gleiche Druckverhältnisse, so daß keine resultierenden Kräfte auf das Sterilisationsgefäß innerhalb der Haltevorrichtung ausgeübt werden.

Durch die weitere Ausgestaltung des Sterilisationsgefäßes nach der zweiten Ausführungsform der Erfindung als napfförmig, ein- oder mehrteilig ausgebildete, rechteckiges Aufnahmegefäß, dessen Längserstreckung quer zur Einschubrichtung liegt, wird das Beschicken weiter vereinfacht. Da die Bodenfläche der Aufnahmewanne fest mit Tragschienen verbunden ist, die hin- und herbeweglich in der Haltevorrichtung gelagert sind, wird eine schubladenähnliche Anordnung erreicht. Infolge der federnd in ihrer unwirksamen Lage gehaltenen Abdeckung, kann das Sterilisationsgefäß leicht in die Haltevorrichtung eingebracht werden. Die Abdeckung ist innerhalb einer austauschbaren Metallfassung in der Haltevorrichtung oberhalb des Sterilisationsgefäßes auf- und abbeweglich angeordnet und als Scheibe ausgebildet die in ihrem Randbereich eine elastische Gummidichtung trägt, die an der inneren Wandung der Metallfassung anliegt. Ferner weist die Abdeckung eine Dampfzuführung in Form eines exzentrisch angeordneten Durchbruchs auf, der mit dem Innenraum des Sterilisationsgefäßes derart kommuniziert, daß zu Beginn des Sterilisationsvorganges die Abdeckung unter dem Druck des einströhmenden Dampfes entgegen der Kraft der Federn gegen die oberen Kanten des Sterilisationsgefäßes drückt wobei die Gummidichtung während des Sterilisationsvorganges sowohl gegenüber der Innenwand der Metallfassung als auch gegenüber dem Sterilisationsgefäß den Sterilisationsraum druckdicht abdichtet. Gleichzeitig wird die Aufnahmewanne und damit das Sterilisationsgefäß in der hierfür vorgesehenen Ausnehmung der Haltevorrichtung infolge der zwischen Sterilisationsgefäß und Haltevorrichtung auftretenden Bremskräfte arretiert.

Zur Erzielung dieser vorteilhaften Wirkung sind die dem Sterilisationsgefäß gemäß der zweiten Ausführungsform zugewandten und die der Ausnehmung der Haltevorrichtung zugewandten Flächen der Abdeckung unterschiedlich groß ausgebildet, so daß infolge des während der Dampfzuführung auftretenden Differenzdruckes die Abdeckung auf das oben offene Sterilisationsgefäß, dieses abdichtend, gepreßt wird. Bei Unterbrechung der Dampfzufuhr wird die Abdeckung von den sie haltenden Federn in ihre unwirksame, das Sterilisationsgefäß freigebende Lage innerhalb der Matellfassung gezogen, so daß das Sterilisationsgefaß frei zugänglich der Haltevorrichtung entnehmbar ist.

Das nach einer weiteren Ausführungsform der Erfindung in Teilgefäße aufgeteilte Sterilisationsgefäß weist besondere bedienungstechnische Vorteile auf, da in jedes dieser Teilgefäße, nach Klassen oder Größen geordnet, ärztliche Instrumente für den Sterilisationsvorgang eingelegt und entnommen werden können.

Die Erfindung ist nachfolgend anhand dreier in der Zeichnung mehr oder minder schematisch dargestellter Ausführungsbeispiele beschrieben.

Es zeigen:
- Figur 1: das Gesamtschema einer Anordnung zum Sterilisieren von ärztlichen Instrumenten und Implantaten mit einem in einer Haltevorrichtung angeordneten Sterilisationsgefäß gemäß dem Stand der Technik,
- Figur 2: eine erste Ausführungsform eines Sterilisationsgefäßes für eine Anordnung nach Figur 1 in perspektivischer Darstellung ohne zugehörige Dichtungen,
- Figur 3: einen Schnitt durch die Haltevorrichtung zur Aufnahme des Sterilisationsgefäßes nach Figur 2,
- Figur 4: eine Seitenansicht der Haltevorrichtung mit aus der Haltevorrichtung herausgezogenem Sterilisationsgefäß, dessen Längserstreckung in der Einschubrichtung liegt,
- Figur 5: eine Seitenansicht der Haltevorrichtung mit vollständig eingeschobenem Sterilisationsgefäß,
- Figur 6: ein Detail betreffend ein Dichtungselement des Sterilisationsgefäßes nach den Figuren 2 bis 5 für die stirnseitigen Wände,
- Figur 7: einen Schnitt durch eine zweite Ausführungsform eines mit seiner Längserstrekkung quer zur Einschubrichtung liegenden Sterilisationsgefäßes in der Arbeitsstellung innerhalb der Haltevorrichtung einer Anordnung nach Figur 1,
- Figur 7a: im vergrößerten Maßstab die Abdeckung des Sterilisationsgefäßes nach Figur 7,
- Figur 8: einen Schnitt durch das Sterilisationsgefäß nach Figur 7 in der das Beschicken mit zu sterilisierenden Instrumenten und Implantaten ermöglichenden Stellung,
- Figur 9: eine Weiterbildung der Ausführungsform des Sterilisationsgefäßes nach Figur 7 im Schnitt und

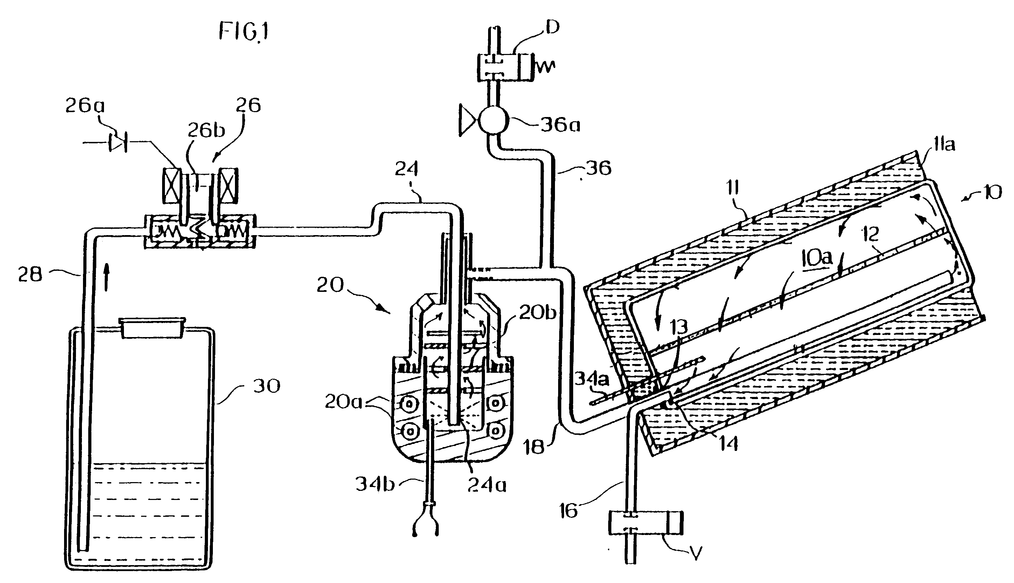

Anstelle eines sonst üblichen Autoklaven wird bei der Anordnung zur Dampf-Sterilisation von ärztlichen Instrumenten und Implantaten nach Figur 1 ein aus Boden- und Deckelteil bestehendes, zur Waagerechten geneigt angeordnetes, als Kassette ausgebildetes Sterilisationsgefäß 10 verwendet, das in einer Ausnehmung einer ortsfest angeordneten Haltevorrichtung 11 eingeschoben werden kann, die eine Wärmeisolierung 11a aufweist. Zur Aufnahme der zu sterilisierenden ärztlichen Instrumente und Implantate weist das Sterilisationsgefäß eine als Lochplatte ausgebildete Aufnahmeplatte 12 auf. Die Haltevorrichtung 11 weist eine durch eine Auslaßöffnung 13 im Sterilisationsgefäß ragende Luft- und Kondensat-Auslaßleitung 16 und eine durch eine Einlaßöffnung 14 in das Sterilisationsgefäß ragende Dampfzuleitung 18 auf, so daß diese im Abstand voneinander in den von dem Sterilisationsgefäß umschlossenen Raum 10a ragen.

Die Luft- und Kondensat-Auslaßleitung 16 ist über ein Ventil V mit Atmosphäre verbindbar, während die Dampfzuleitung 18 mit einem Dampfgenerator 20 verbunden ist. Die Dampfkammer des Dampfgenerators 20 wird über elektrische Heizelemente 20a aufgeheizt und über eine Speiseleitung 24, die an ihrem Ende Austrittsöffnungen 24a aufweist, pulsartig mit Wasser versorgt, wobei über im Innern der Dampfkammer angeordnete Prallplatten 20b ein direktes Einspritzen von siedendem Wasser in die Dampfzuleitung 18 verhindert wird.

In die den Dampfgenerator mit Wasser versorgende Speiseleitung 24 ist eine Dosierpumpe 26 eingefügt, die über eine Saugleitung 28 mit einem Behälter 30 kommuniziert, der destilliertes Wasser enthält. Der Antrieb der Dosierpumpe 26 erfolgt über einen von einem über eine Diode 26a gespeisten Elektromagneten betätigten Kolben 26b. Über Thermofühler 34a und 34b werden die Temperaturen in der Dampfkammer des Dampfgenerators und in dem Sterilisationsgefäß gemessen.

Schließlich kann die Sterilisationskammer über eine Abzweigleitung 36 und einen Druckregler 36a mit einer nicht dargestellten, über ein Ventil D anschaltbaren Druckluftquelle verbunden werden, um am Ende eines Sterilisationsvorganges die Instrumente und Implantate zu kühlen und zu trocknen.

Über eine nicht dargestellte Steuereinrichtung wird der Ablauf eines Sterilisationsvorganges - bei dem der aus der Dampfzuleitung austretende Dampfstoß schwallartig das Sterilisationsgefäß durchläuft - gesteuert. Diese und das hierzu gehörende Verfahren für den Betrieb der beschriebenen Anordnung bilden nicht den Gegenstand der Erfindung und sind beispielsweise in der EP 0 429 960 A2 dargestellt und beschrieben.

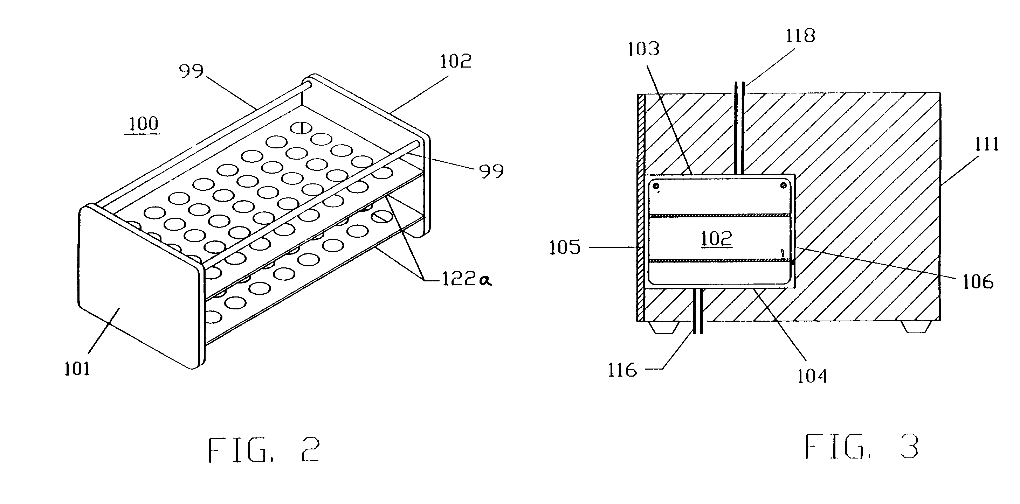

Nunmehr sei in Verbindung mit den Figuren 2 bis 6 das erste Ausführungsbeispiel des erfindungsgemäßen Sterilisationsgefäßes 100 beschrieben.

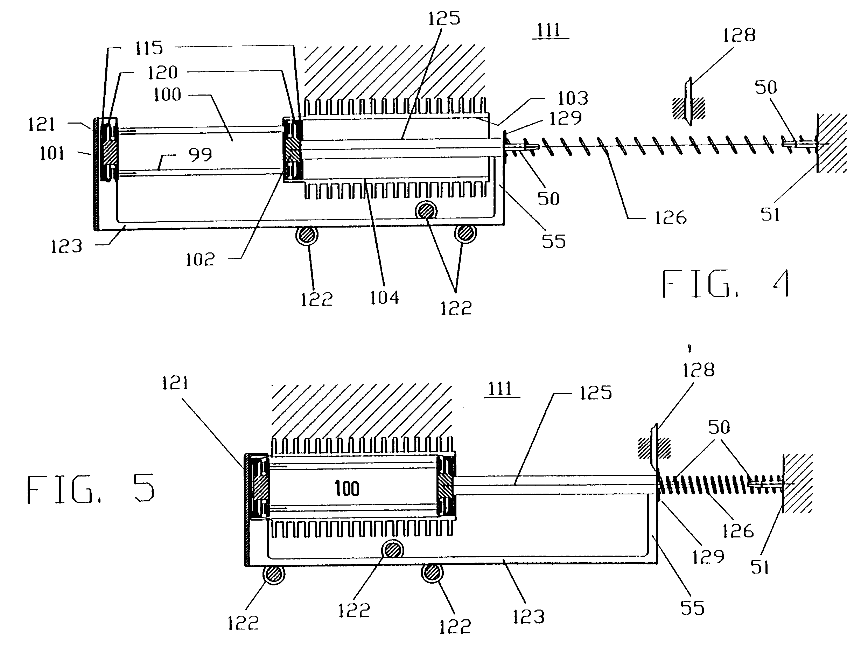

Wie Figur 2 zeigt, umfaßt das Sterilisationsgefäß 100 lediglich eine Vorder- und eine Rückwand 101 und 102, die über Verbindungselemente, z.B. mittels Bolzen 99, als auch über einen oder mehrere Zwischenböden 122 miteinander fest verbunden sind, die zur Aufnahme der zu sterilisierenden Implantate und Instrumente als Lochplatten ausgebildet sind. Die vier Seitenwände für dieses Sterilisationsgefäß werden von den eine quaderförmige Ausnehmung in der Haltevorrichtung für die Aufnahme des in Einschubrichtung sich längs erstreckenden Sterilisationsgefäßes einschließenden Wandungen 103, 104, 105 und 106 der Haltevorrichtung 111 gebildet, wie dies aus Figur 3 deutlich ersichtlich ist. Demgemäß sind dem Sterilisationsgefäß lediglich die Vorder- und die Rückwand zugeordnet, während die, eine lediglich an einer Stirnseite offene Ausnehmung bildenden Seitenwandungen sowie die Ober- und Unterwandung der Haltevorrichtung zugeordnet sind.

Im Gegensatz zur Ausbildung nach Figur 1 mündet hier die Dampfzuleitung 118 im oberen Bereich nahe der Rückwand 102, also in die Wandung 103 der Haltevorrichtung, während der Luft- und Kondensat-Auslaß 116 im unteren Bereich nahe der Vorderwand 101, also in die Wandung 104 der Haltevorrichtung, mündet, so daß auch hier der austretende Dampfstoß schwallartig das Sterilisationsgefäß durchströmt.

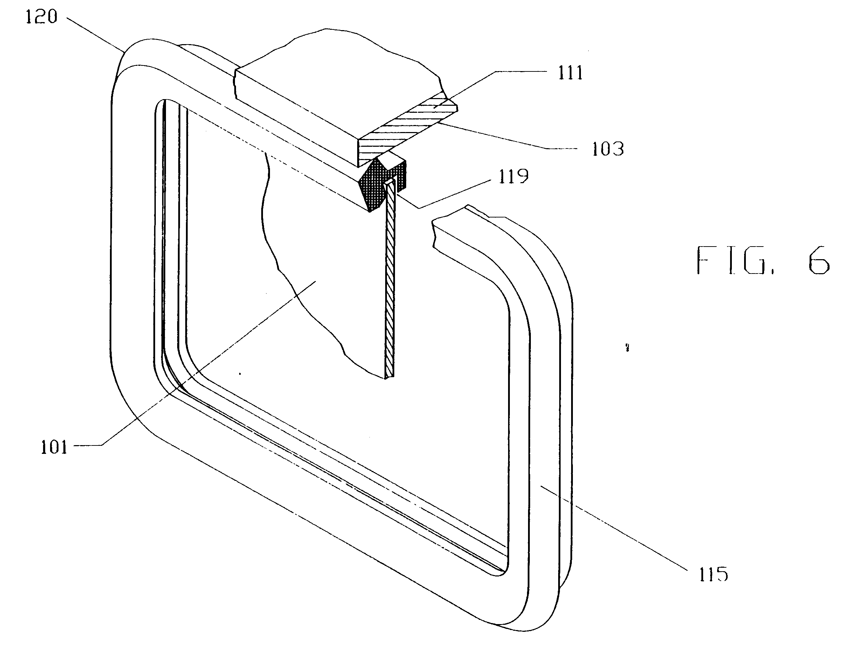

Zur Abdichtung des innerhalb der Kaltevorrichtung in vorstehend beschriebener Weise gebildeten Sterilisationsraumes dienen den Stirnseiten, also der Vorder- und Rückwand des Sterilisationsgefäßes 100 zugeordnete, mit der Vorder- und Rückwand 101 und 102 austauschbar verbundene, untereinander gleichartig ausgebildete, aus Kunststoff oder Gummi bestehende Dichtungen 115. Jede Dichtung 115 weist einen lippenförmigen, nach außen gerichteten Rand 120 sowie eine die Randbereiche von Vorder- bzw. Rückwand umfassende Nut 119 auf, wie dies insbesondere der Figur 6 zu entnehmen ist.

Der Vorderwand 101 des Sterilisationsgefäßes ist eine Abdeckkappe 121 zugeordnet, die im eingeschobenen Zustand des Sterilisationsgefäßes die Ausnehmung in der Haltevorrichtung 111 verdeckt, um ein gefälliges Aussehen der Haltevorrichtung zu gewährleisten.

Diese Abdeckkappe ist Teil einer dem Sterilisationsgefäß 100 zugeordneten, als Tropfgefäß ausgebildeten Führungsvorrichtung 123, die mittels Rollen 122 in der Haltevorrichtung hin- und herbeweglich gelagert ist.

Das hintere Ende der Führungsvorrichtung 123 bildet eine Traverse 55, die über einen Stab 125 mit der Rückwand 102 des sterilisationsgefäßes 100 starr verbunden ist.

Schließlich ist, wie in Figuren 4 und 5 dargestellt, eine auf, von einer Rückwand 51 der Haltevorrichtung 111 und von dem Stab 125 sich erstreckenden Fortsätzen 50 aufgesetzte Feder 126 in der Haltevorrichtung 111 vorgesehen, die beim Entspannen das Überführen des Sterilisationsgefäßes 100 in die in Figur 4 dargestellte Offenstellung bewirkt. Um das eingeschobene Sterilisationsgefäß in der eingeschobenen, die Feder 126 spannende Lage zu halten, ist eine, in hier nicht dargestellter Weise, elektromagnetisch oder über ein Druckmittelstellglied betätigbare Verriegelung in Form eines Anschlages 128 vorgesehen, der hinter einen korrespondierenden Anschlag 129 der Haltevorrichtung greift, sobald dieses in die in Figur 5 dargestellte, vollständig eingeschobene Lage bewegt ist.

Im eingeschobenen Zustand legen sich während eines Sterilisationsvorganges die über den Umfangsbereich - Vorder- und Rückwand - des Sterilisationsgefäßes ragenden lippenförmigen Ränder 120 der Dichtung 115 unter dem dann dort herrschenden Dampfdruck an die Wandungen 103/104 und 105/106 der Haltevorrichtung dichtend an und halten dabei das Sterilisationsgefäß in seiner eingeschobenen Lage in der Haltevorrichtung dicht; vgl. Figur 5.

Schließlich kann eine an sich bekannte Dämpfungsvorrichtung am Sterilisationsgefäß angreifen, die die durch die Feder 126 bewirkte schlagartige Ausstoßbewegung des Sterilisationsgefäßes in eine kontinuierlich ablaufende Austrittsbewegung umformt.

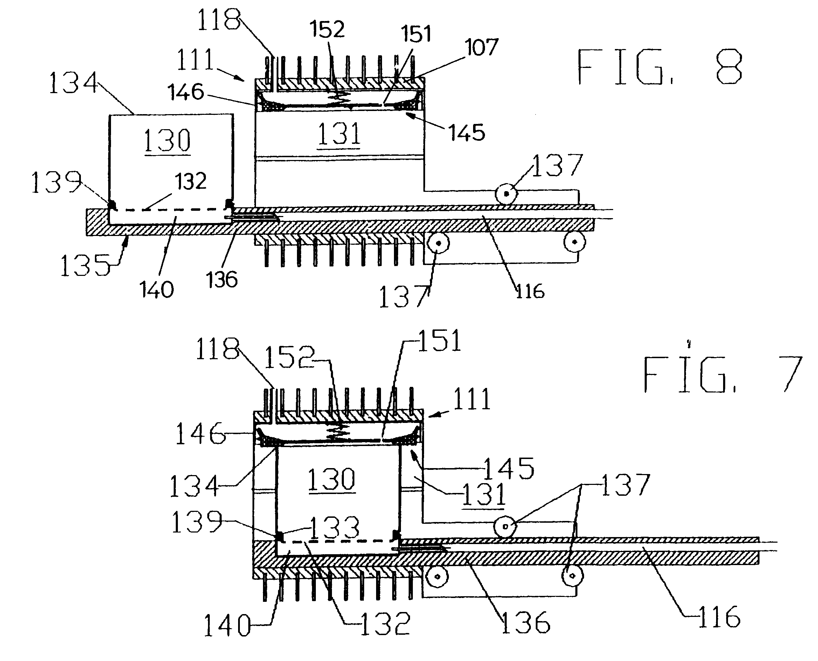

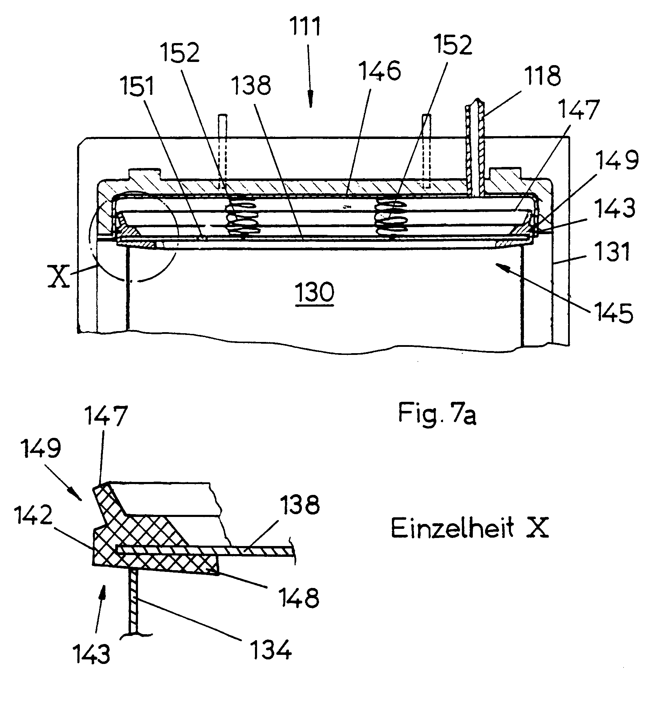

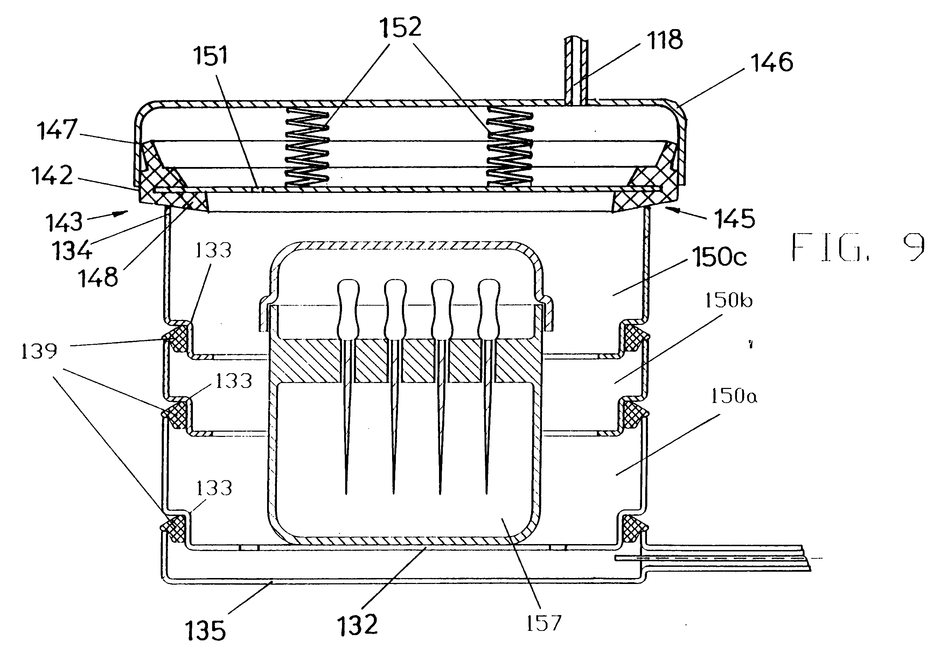

Nach einer weiteren Ausführungsform des insgesamt mit der Bezugsziffer 130 bezeichneten Sterilisationsgefäßes gemäß den Figuren 7, 7a und 8 ist dieses als quaderförmiger Napf, also mit Vorder- und Rückwand sowie mit Seitenwänden und einer gelochten Bodenfläche 132 ausgebildet. Das Sterilisationsgefäß 130 weist, seiner gelochten Bodenfläche 132 benachbart, einen eingezogenen Rand 133 auf und ist oben offen. Der Bodenfläche 132 ist eine, dem Umfang des Sterilisationsgefäßes angepaßte, rechteckig geformte, nach unten geschlossene, schalenförmige Aufnahmevorrichtung 135 zwecks lösbarer Aufnahme des Sterilisationsgefäßes 130 zugeordnet. Diese schalenförmig ausgebildete Aufnahmevorrichtung ist mittels einer Schienenführung 136, deren Tragschienen zwischen in der Haltevorrichtung 111 gelagerten Rollen 137 geführt sind, von einer außerhalb der Haltevorrichtung befindlichen Aufnahmestellung (Figur 8) in eine innerhalb der Haltevorrichtung in einer hierfür vorgesehenen Ausnehmung 131 befindliche Arbeitsstellung (Figur 7) und vice versa überführbar. Zwischen Aufnahmevorrichtung 135 und eingezogenem Rand 133 ist ringsum eine Dichtung 139 angeordnet, die die gelochte Bodenfläche 132 des sterilisationsgefäßes in der in die Aufnahmevorrichtung 135 eingesetzten Lage gegenüber Atmosphäre abdichtet.

Das mit seiner Längserstreckung quer zur Einschubrichtung liegende Sterilisationsgefäß nach dieser Ausführungsform ist also nach Art einer vollständig ausziehbaren Schublade in der hierfür vorgesehenen Ausnehmung 131 der Haltevorrichtung 111 angeordnet, deren in Bezug auf die Figuren 7 und 8 obere Wandung 107 in noch zu beschreibender Weise die hier fehlende Wandung des Sterilisationsgefäßes 130 bildet.

Im schalenförmigen Teil der Aufnahmevorrichtung 135 mündet der Luft- und Kondensat-Auslaß 116 in Form eines Rohres, das mit dem Raum 140 unterhalb der Bodenfläche 132 der Aufnahmevorrichtung 135 kommuniziert.

Die Abdeckung des Sterilisationsgefäßes erfolgt - wie insbesondere Figur 7a zeigt - über einen rechteckig ausgebildeten Deckel - oder Abdeckung - 145, der ähnlich einem Kolben in einer rahmenförmigen Metallfassung 146 innerhalb der das Sterilisationsgefäß 130 aufnehmenden Ausnehmung 131 in der Haltevorrichtung 111 ortsfest, aber auf- und abbeweglich über Federn 152 an der Metallfassung 146 aufgehängt ist. Der Deckel 145 überragt den oberen offenen Bereich, also die oberen Kanten 134 des Sterilisationsgefäßes 130 rundum allseits und trägt ein Dichtprofil 143, das im Querschnitt etwa L-förmig ist, wobei der untere Schenkel 148 langgestreckt ist zwecks Aufnahme einer den Deckel bildenden Scheibe 138 und Abdeckung der oberen Kanten 134 des Sterilisationsgefäßes, wie dies insbesondere die Einzelheit X zeigt, während der in Bezug auf Figur 7a außenliegende Schenkel 149 eine Führungsfläche 142 bildend sich an der inneren Wandung der Metallfassung 146 anlegt. Eine Lippe 147 des Schenkels 149 bildet dabei eine weitere Dichtfläche des Dekkels 145. Die untere Fläche des Schenkels 148 des im Querschnitt etwa L-förmigen Dichtprofils liegt im Betriebszustand auf den Kanten 134 des Sterilisationsgefäßes 130 dichtend auf. Der Deckel weist eine exzentrische Öffnung 151 auf, die diesen durchdringt und den vom Sterilisationsgefäß umschlossenen Raum mit dem freien Raum oberhalb des Deckels innerhalb der Metallfassung 146 verbindet.

Die Dampfzuleitung 118 mündet hier oberhalb des Deckels in die das Sterilisationsgefäß 130 aufnehmende Ausnehmung 131 der Haltevorrichtung 111. Da die abgedeckte Fläche des Sterilisationsgefäßes kleiner als die freie, der Dampfzuführungsleitung 118 zugewandte Fläche des Deckels ist, wird der Deckel unter Dampfdruck infolge des dabei auftretenden Differenzdruckes gegen die Oberkanten 134 des Sterilisationsgefäßes entgegen der Wirkung der Federn 152 bewegt und hält den Innenraum des Sterilisationsgefäßes druckdicht verschlossen. Diese Druckdifferenz zwischen Ober- und Unterfläche des Dekkels hält gleichzeitig auch das Sterilisationsgefäß in der eingeschobenen Lage in der Ausnehmung infolge der bei diesem Dichtvorgang auftretenden Bremskräfte zwischen Sterilisationsgefäß und Haltevorrichtung und Gehäuse.

Bei Wegfall des Dampfdruckes ziehen die Federn 152 den Deckel in seine Ruhelage innerhalb der Metallfassung 146 und das Sterilisationsgefäß 130 wird freigegeben.

Auch bei dieser Ausführungsform ist eine, das Ausstoßen des Sterilisationsgefäßes bewirkende Druckfeder 126 vorgesehen, wie dies in Verbindung mit den Figuren 4 und 5 bereits beschrieben ist.

Eine weitere Ausbildung des Sterilisationsgefäßes ist in Figur 9 dargestellt, unter Verwendung der gleichen Haltevorrichtung, wie sie für das Ausführungsbeispiel nach den Figuren 7 bis 8 beschrieben worden ist.

Anstelle eines integralen Sterilisationsgefäßes ist hier eine Vielzahl von jeweils den gleichen Grundriß aufweisenden Teilgefäßen 150a, 150b und 150c (Norm Tray) vorgesehen, wobei der zur Verfügung stehende Höhenbereich der Ausnehmung 131 der Haltevorrichtung 111 in drei Teilbereiche unterteilt ist. Die Teilgefäße sind stapelfähig ausgebildet. Hierzu weist jedes der Teilgefäße 150a, 150b und 150c ebenfalls einen eingezogenen, mit dem oberen Randbereich des zugeordneten Teilgefäßes korrespondierenden Randbereich 133 auf, in dem jeweils eine Dichtung 139 angeordnet ist. Die unterste Dichtung eines jeden Stapels von Teilgefäßen ist hier ebenfalls der Aufnahmevorrichtung 135 zugeordnet, wie es beim Ausführungsbeispiel nach den Figuren 7 bis 8 beschrieben und dargestellt ist. Der offene Flächenbereich der zu oberst liegenden Teilgefäße wird hier ebenfalls mittels des in Verbindung mit den Figuren 7 bis 8 im Detail beschriebenen rechteckförmigen Deckels - Abdeckung - 145 während eines Sterilisationsvorganges druckdicht verschlossen, so daß auch hier die gesamte Anordnung von Teilgefäßen während der Sterilisationsvorgänge gegenüber Atmosphäre abgedichtet und in der Ausnehmung der Haltevorrichtung festgehalten sind. Die Bodenflächen der einzelnen Teilgefäße weisen Lochungen für den Dampfdurchlaß auf. Ebenso erfolgt hier die Dampfzuführung über die Öffnung 151 in dem Deckel und die Luft- und Kondensatabführung über das Rohr 116 im Bereiche der Schienenführung 136.

Wie die Figur 9 ferner zeigen sind z.B. rechteckige, miteinander fluchtende Durchbrüche in den Bodenflächen 132 der Teilgefäße 150 vorgesehen, die einen über mehrere Teilgefäße sich erstreckenden Aufnahmeraum für in einem Ständer 157 angeordnete Instumente und/oder Implantate bilden.

Ferner kann den Teilgefäßen ein nach Art eines Deckels ausgebildetes Teilgefäß zugeordnet sein, dessen Bodenfläche 132 durchbrochen ist.

Durch dieses Teilgefäß kann unbeabsichtigte Berührung sterilen Gutes verhindert werden.

## Patentansprüche

1. Anordnung zur Dampf-Sterilisation von ärztlichen Instrumenten, Implantaten und ähnlichem, mit einem von einer Dosierpumpe gespeisten Dampfgenerator (30), einem Sterilisationsgefäß (100, 130, 150), das in einer mit dem Dampfgenerator in Wirkverbindung stehenden Haltevorrichtung (111) einschiebbar angeordnet ist, und mit einer Steuereinrichtung für den Sterilisationsvorgang, **dadurch gekennzeichnet, daß** die Wandungen des während des Sterilisationsvorganges allseits druckdicht geschlossenen Sterilisationsgefäßes (100, 130, 150) teilweise von Teilen der Haltevorrichtung (111) gebildet sind.

2. Anordnung nach Anspruch 1, **dadurch gekennzeichnet, daß** das Sterilisationsgefäß auch während des Beschickens mit den zu sterilisierenden Instrumenten und Implantaten mit der zugeordneten Haltevorrichtung betrieblich verbunden bleibt.

3. Anordnung nach Anspruch 1, **dadurch gekennzeichnet, daß** dem Sterilisationsgefäß (100) lediglich die Vorder- und die Rückwand (101, 102) zugeordnet sind, während die Seiten-, sowie die Ober- und Unterwandung (103 bis 106) der Haltevorrichtung (111) zugeordnet sind, wobei mindestens eine die Vorder- und Rückwand (101, 102) verbindende Lochplatte (112) der Aufnahme der zu sterilisierenden Instrumente und Implantate dient.

4. Anordnung nach Anspruch 1, **dadurch gekennzeichnet, daß** der Vorder- und Rückwand (101, 102) jeweils eine deren Randbereiche umfassende (Nut 119) Dichtung (115) zugeordnet ist, die im eingeschobenen Zustand des Sterilisationsgefäßes (100) den von den genannten Wänden (103 bis 106) eingeschlossenen Raum der Haltevorrichtung (111) gegenüber Atmosphäre abdichtet.

5. Anordnung nach Anspruch 1, **dadurch gekennzeichnet, daß** Unter- und Oberseite (132, 134) des Sterilisationsgefäßes (130) je eine Dichtung (139, 145) zugeordnet ist, die im eingeschobenen Zustand des Sterilisationsgefäßes (100) den von der Haltevorrichtung eingeschlossenen Raum (131) gegenüber Atmosphäre abdichtet.

6. Anordnung nach den Ansprüchen 1 bis 5, **dadurch gekennzeichnet, daß** eine mit der Haltevorrichtung (111) verschieblich gelagert verbundene Halte- oder Aufnahmevorrichtung (111, 135) für die lösbare Aufnahme des Sterilisationsgefäßes (100, 130) vorgesehen ist.

7. Anordnung nach Anspruch 1, **dadurch gekennzeichnet, daß** in den Sterilisationsgefäßraum eine in der Haltevorrichtung (111) fest installierte Dampfzuleitung (118) sowie eine Luft- und Kondensat-Abführleitung (116) münden.

8. Anordnung nach den Ansprüchen 1 bis 6, **dadurch gekennzeichnet, daß** die mit der Haltevorrichtung (111) verschiebbar verbundene Stützvorrichtung (123) als Auffangwanne für abtropfende Feuchtigkeit der den Sterilisationsraum verlassenden ärztlichen Instrumente und Implantate ausgebildet ist.

9. Anordnung nach Anspruch 1, **dadurch gekennzeichnet, daß** dem Sterilisationsgefäß (100, 130, 150) eine das Ausstoßen bewirkende Druckfeder (126) zugeordnet ist, der für das Auslösen des Ausstoßvorganges des Sterilisationsgefäßes eine druck- oder elektromagnetisch betätigte Verriegelungsvorrichtung (128, 129) zugeordnet ist.

10. Anordnung nach einem oder mehreren der vorhergehenden Ansprüche, **dadurch gekennzeichnet, daß** das Sterilisationsgefäß (130) als napfförmiges, rechteckiges Aufnahmegefäß ausgebildet ist, dessen Längserstreckung quer zur Einschubrichtung liegt, und der eine in der Haltevorrichtung (111) befindliche schienengeführte (136) Aufnahmevorrichtung (135) zugeordnet ist, in der die Luft- und Kondensat-Abführleitung (116) angeordnet ist.

11. Anordnung nach Anspruch 5, **dadurch gekennzeichnet, daß** eine federnd in ihrer unwirksamen Lage gehaltene Abdeckung (145) mit einer Scheibe (138) als Träger einer ihren Randbereich bildenden elastischen Gummidichtung (143) vorgesehen ist, die innerhalb einer austauschbaren Metallfassung (146) in der Haltevorrichtung (111) oberhalb des Sterilisationsgefäßes (130) auf- und abbeweglich angeordnet ist, daß die dem Sterilisationsgefäß (130, 150) zugewandten und die der Ausnehmung (131) der Haltevorrichtung zugewandten Flächen der Abdeckung (145) unterschiedlich groß ausgebildet sind, und daß die Abdeckung (145) eine Dampfzuführung in Form eines exzentrisch angeordneten Durchbruchs (Öffnung 151) aufweist, der mit dem Innenraum des Sterilisationsgefäßes (130) kommuniziert.

12. Anordnung nach Anspruch 11, **dadurch gekennzeichnet, daß** die Gummidichtung (142) im Querschnitt etwa L-förmig ausgebildet ist, deren die Basis bildende Schenkel (148) die Scheibe (138) der Abdeckung (145) umfassen und eine Dichtfläche bilden, während die anderen Schenkel (149) eine an der seitlichen Innenfläche der Metallfassung (146) anliegende Führungsfläche und eine Dichtungslippe (147) aufweisen.

13. Anordnung nach Anspruch 12, **dadurch gekennzeichnet, daß** das Sterilisationsgefäß (150) in Dichtungen (139) tragende Teilgefäße (150a, 150b, 150c) mit gleichen Grundflächen aufgeteilt ist und deren Höhenabmessungen derart gewählt sind, daß alle Teilgefäße mittels des gleichen Deckels (145) innerhalb der Ausnehmung (131) der Haltevorrichtung (111) druckdicht verschließbar sind.

## Claims

1. Assembly for the steam sterilisation of surgical instruments, implants and the like, with a steam generator (30) fed from a dosing pump, a sterilisation vessel (100, 130, 150) arranged to be insertable in a holding device (111), operatively connected with the steam generator, and with a control device for the sterilisation process, characterised by the fact that the walls of the sterilisation vessel (100, 130, 150) which are pressure sealed on all sides during the sterilisation process, are to some extent formed by parts of the holding device (111).

2. Assembly according to Claim 1, characterised by the fact that the sterilisation vessel remains operatively connected with the associated holding device while being fed with the instruments and implants to be sterilised.

3. Assembly according to Claim 1, characterised by the fact that only the front wall and rear wall (101, 102) are associated with the sterilisation vessel (100), while the side walls and also the upper and lower walls (103 to 106) are associated with the holding device (111), while at least one perforated plate (112) interconnecting the front and rear wall (101,102) serves to accommodate the instruments and implants to be sterilised.

4. Assembly according to Claim 1, characterised by the fact that the front wall and rear wall (101,102) are in each case provided with a grooved (119) seal (115) which surrounds the edge zones of the said walls and by which, when the sterilisation vessel (100) has been inserted the volume of the holding device (111) enclosed by the said walls (103 to 106) is sealed off against the atmosphere.

5. Assembly according to Claim 1, characterised by the fact that the lower and upper side (132,134) of the sterilisation vessel (130) are in each case provided with a seal (139, 145) by which, with the sterilisation vessel (100) inserted, the volume (131) of the holding device is sealed off from the atmosphere.

6. Assembly according to Claim 1 to 5, characterised by the fact that a holding or receiver device (111,135) movably connected with the holding device (111) is provided to accommodate the sterilisation vessel (100,130) in a releasable manner.

7. Assembly according to Claim 1, characterised by the fact that a steam supply pipe (118) permanently mounted in the holding device (111) and also an air and condensate discharge pipe (116) lead into the sterilisation chamber.

8. Assembly according to Claim 1 to 6, characterised by the fact that the supporting device (123) movably connected with the holding device (111) is constructed as a collector vessel for liquid dripping off the surgical instruments and implants leaving the sterilisation chamber.

9. Assembly according to Claim 1, characterised by the fact that a pressure spring (126) which actuates the ejection is associated with the sterilisation vessel (100,130) and is provided with a pressure operated or electromagnetically operated locking device (128,129) in order to initiate ejection of the sterilisation vessel.

10. Assembly according to any one of the preceding claims, characterised by the fact that the sterilisation vessel (130) takes the form of a rectangular pot-shaped receiver vessel of which the length extends transversally to the insertion direction and with which is associated a receiver device (135) which is guided on rails (136) and which is situated in the holding device (111) and in which the air and condensate discharge pipe (116) is positioned.

11. Assembly according to Claim 5, characterised by the fact that a covering (145) elastically secured in an idle position is provided with a plate (138) as a support for a rubber packing (143) which forms the edge zone and which is mounted to be movable up and down within a removable and replaceable metal mounting (146) in the holding device (111) above the sterilisation vessel (130), those surfaces of the covering device (145) which face towards the sterilisation vessel (130,150) and those which face towards the recess (131) of the holding device being of a different size, and the covering device (145) being provided with a steam supply orifice in the form of an eccentrically positioned perforation (aperture 151) communicating with the interior of the sterilisation vessel (130).

12. Assembly according to Claim 11, characterised by the fact that the rubber packing (142) has an approximately L-shaped cross section of which the sides forming the base (148) surround the plate (138) of the covering (145) and form a sealing surface while other sides (149) are provided with a guide surface resting against the lateral internal surface of the metal mounting (146) and also with a sealing lip (147).

13. Assembly according to Claim 12, characterised by the fact that the sterilisation vessel (150) is subdivided into part-vessels (150a, 150b, 150c) provided with seals (139) and having equal base areas and constructed to heights which ensure that all part-vessels can be closed in a pressure-proof manner by means of one and the same cover (145) within the recess (131) of the holding device (111).

## Revendications

1. Agencement pour la stérilisation par la vapeur d'instruments médicaux, d'implants et analogues, comprenant un générateur de vapeur (30) alimenté par une pompe doseuse, un récipient de stérilisation (100, 130, 150) disposé avec la possibilité d'être inséré dans un dispositif de retenue (111) en liaison active avec le générateur de vapeur, et un dispositif de commande du processus de stérilisation, caractérisé en ce que les parois du récipient de stérilisation (100, 130, 150) fermé de toutes parts de manière étanche à la pression sont formées en partie par des parties du dispositif de retenue (111).

2. Agencement selon la revendication 1, caractérisé en ce que le récipient de stérilisation reste en liaison opérationnelle avec le dispositif de retenue associé, même au cours de son chargement avec les instruments et implants à stériliser.

3. Agencement selon la revendication 1, caractérisé en ce que seules les parois avant et arrière (101, 102) sont associées au récipient de stérilisation (100) tandis que les parois latérales ainsi que les parois de dessus et de dessous (103 à 106) le sont au dispositif de retenue (111), au moins une plaque perforée (112) qui relie les parois avant et arrière (101, 102) servant à recevoir les instruments et implants à stériliser.

4. Agencement selon la revendication 1, caractérisé en ce qu'à chacune des parois avant et arrière (101, 102) est associé un joint (115) qui entoure leur zone de bord (gorge 119) et qui, lorsque le récipient de stérilisation (100) est en position insérée, rend étanche par rapport à l'atmosphère l'espace du dispositif de retenue (111) défini par lesdites parois (103 à 106).

5. Agencement selon la revendication 1, caractérisé en ce qu'au dessous et au dessus (132, 134) du récipient de stérilisation (130) est respectivement associé un joint (139, 145) qui, lorsque le récipient de stérilisation (100) est en position insérée, rend étanche par rapport à l'atmosphère l'espace (131) défini par le dispositif de retenue.

6. Agencement selon les revendications 1 à 5, caractérisé en ce qu'un dispositif de retenue ou de réception (111, 135) relié de manière coulissante au dispositif de retenue (111) est prévu pour recevoir de manière amovible le récipient de stérilisation (100, 130).

7. Agencement selon la revendication 1, caractérisé en ce qu'une conduite d'arrivée de vapeur (118) installée à demeure dans le dispositif de retenue (111) ainsi qu'une conduite d'évacuation d'air et de condensation (116) débouchent dans l'espace du récipient de stérilisation.

8. Agencement selon les revendications 1 à 6, caractérisé en ce que le dispositif porteur (123) relié de manière coulissante au dispositif de retenue (111) est conçu sous la forme d'un bac collecteur pour les gouttes d'humidité tombant des instruments médicaux et des implants qui quittent l'espace de stérilisation.

9. Agencement selon la revendication 1, caractérisé en ce qu'au récipient de stérilisation (100, 130, 150) est associé un ressort de compression à effet d'éjection (126) auquel est associé un dispositif de verrouillage (128, 129) actionné par pression ou par électro-aimant pour déclencher le processus d'éjection du récipient de stérilisation.

10. Agencement selon une ou plusieurs des revendications précédentes, caractérisé en ce que le récipient de stérilisation (130) est conçu sous la forme d'un récipient récepteur rectangulaire en forme de cuvette dont l'extension longitudinale est transversale au sens d'insertion et auquel est associé un dispositif récepteur (135) sur rail (136), qui se trouve dans le dispositif de retenue (111) et dans lequel est disposée la conduite d'évacuation d'air et de condensation (116).

11. Agencement selon la revendication 5, caractérisé en ce qu'il est prévu un couvercle (145) qui est maintenu élastiquement en position inactive et est muni d'une plaque (138) pour porter un joint en caoutchouc élastique (143) formant sa zone de bord et qui est disposé avec la possibilité de monter et de descendre à l'intérieur d'un châssis métallique échangeable (146) dans le dispositif de retenue (111) au-dessus du récipient de stérilisation (130), en ce que les surfaces du couvercle (145) tournées vers le récipient de stérilisation (130, 150) et celles tournées vers la cavité (131) du dispositif de retenue sont de différentes tailles, et en ce que le couvercle (145) comporte une arrivée de vapeur en forme de passage excentré (orifice 151) qui communique avec l'espace intérieur du récipient de stérilisation (130).

12. Agencement selon la revendication 11, caractérisé en ce que le joint en caoutchouc (142) présente une section transversale sensiblement en forme de L dont la branche (148) formant la base entoure la plaque (138) du couvercle (145) et forme une portée de joint, et dont l'autre branche (149) comporte une surface de guidage appliquée contre la surface intérieure latérale du châssis métallique (146) et une lèvre d'étanchéité (147).

13. Agencement selon la revendication 12, caractérisé en ce que le récipient de stérilisation (150) est divisé en récipients partiels (150a, 150b, 150c) qui portent des joints (139) et présentent des surfaces de base identiques et dont les dimensions en hauteur sont choisies pour que tous les récipients partiels puissent être fermés de manière étanche à la pression par le même couvercle (145) à l'intérieur de la cavité (131) du dispositif de retenue (111).
